# EUROPÄISCHE PATENTSCHRIFT

(11) **EP 1 309 576 B1**
(45) Veröffentlichungstag und Bekanntmachung des Hinweises auf die Patenterteilung: **22.11.2017**
(21) Anmeldenummer: 01969223.5
(22) Anmeldetag: 07.08.2001
(51) Int. Cl.: C07D 277/42, C07D 513/04, C08G 75/32, G03G 5/06, H01L 51/00

(54) **DI(HET)ARYLAMINOTHIAZOL-DERIVATE UND IHRE VERWENDUNG IN ORGANISCHEN LICHTEMITTIERENDEN DIODEN (OLEDS) UND ORGANISCHEN PHOTOVOLTAISCHEN BAUELEMENTEN**
DI(HET)ARYLAMINOTHIAZOLE DERIVATIVES AND THEIR USE IN ORGANIC LIGHT-EMITTING DIODES (OLED'S) AND ORGANIC PHOTOVOLTAIC COMPONENTS
DERIVES DE DI(HET)ARYLAMINOTHIAZOL ET LEUR UTILISATION DANS DES DIODES ELECTROLUMINESCENTES ORGANIQUES (DELO) ET COMPOSANTS PHOTOVOLTA QUES ORGANIQUES

(30) Priorität: 07.08.2000 DE 10038437
(43) Veröffentlichungstag der Anmeldung: 14.05.2003
(73) Patentinhaber: OSRAM OLED GmbH, 93049 Regensburg (DE)
(72) Erfinder: HARTMANN, Horst, 06217 Merseburg (DE); KANITZ, Andreas, 91315 Höchstadt (DE); ROGLER, Wolfgang, 91096 Möhrendorf (DE); SCHUMANN, Jörg, 91056 Erlangen (DE)
(74) Vertreter: Epping - Hermann - Fischer
(86) Internationale Anmeldenummer: PCT/DE2001/003016
(87) Internationale Veröffentlichungsnummer: WO 2002/012212

(56) Entgegenhaltungen:
- EP-A- 0 866 110
- DE-A- 4 122 563
- GB-A- 1 185 327
- US-A- 3 467 666
- DATABASE WPI Section Ch, Week 198901 Derwent Publications Ltd., London, GB; Class A14, AN 1989-003179 XP002186877 -& JP 63 280258 A (MITSUBISHI PAPER MILLS LTD), 17. November 1988 (1988-11-17)

## Beschreibung

Die Erfindung betrifft neue Di(het)arylaminothiazol-Derivate, d.h. Diarylaminothiazol- bzw. Dihetarylaminothiazol-Derivate ("hetaryl" = "heteroaryl"), sowie deren Herstellung und Verwendung.

Für organische lichtemittierende Dioden (organische LEDs = OLEDs) und organische photovoltaische Bauelemente werden organische Materialien benötigt, die zur Elektrolumineszenz befähigt sind. Dies können entweder Verbindungen mit geringer Molekülgröße sein (siehe beispielsweise US-PS 4 539 507), die verdampfbar sind, oder polymere Materialien (siehe beispielsweise US-PS 5 247 190), die durch Spin-coating verarbeitet werden können.

In EP 0866110 A1 werden Licht emittierende Materialien offenbart.

Die JP 63280255 A offenbart Thiazol-Derivate für den Einsatz in einer elektrographischen Vorrichtung.

Die Synthese von Verbindungen der genannten Art erfordert Aromatenkupplungsreaktionen. Derartige Reaktionen, bei denen halogenhaltige Verbindungen eingesetzt werden, verlaufen zum Teil unter Metallkatalyse, beispielsweise nach einer Heck-Reaktion oder nach einer Suzuki-Reaktion (siehe dazu: "Chem. Commun.", 1999, Seiten 1837 bis 1838). Hierbei ist es aber kaum möglich, das Metall, wie Palladium (siehe dazu: "Römpp Chemie-Lexikon", 9. Auflage, Seite 1750), vollständig zu entfernen. Metalle - und auch Spuren von nicht vollständig umgesetzten halogenhaltigen Zwischenprodukten - wirken aber als sogenannte Quencher, d.h. sie löschen in ihrer Umgebung die Elektrolumineszenz, und sie setzen deshalb die Effizienz der hergestellten Materialien stark herab.

Bei einer Aromatenkupplung entsprechend einer Ullmann-Reaktion (siehe dazu: "Römpp Chemie-Lexikon", 9. Auflage, Seite 4796) ist die Bildung von Nebenprodukten und von Crackprodukten, die infolge hoher Prozesstemperaturen entstehen, ein großes Problem. Hierbei ist nämlich die Reinigung des Reaktionsproduktes in vertretbarem Ausmaß kaum oder überhaupt nicht möglich.

Die Erfindung erweitert die Materialpalette der Ladungstransportmaterialien insbesondere für den Aufbau von Ladungstransportkaskaden und leitet sich aus dem Patent DE 10002424.6 ab.

Gegenstand der Erfindung sind deshalb 4-substituierte 2-(N,N-Di(het)arylamino)-thiazol-Derivate der folgenden allgemeinen Strukturen VIe, VIm, VIp und VIt: 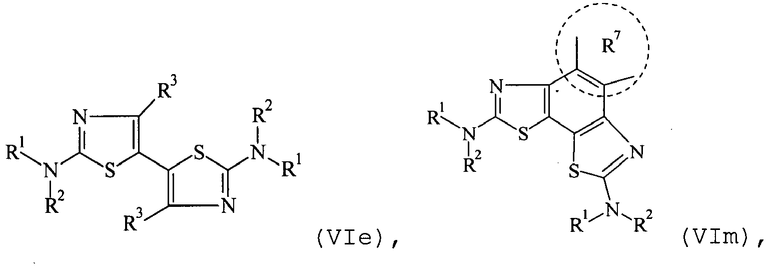 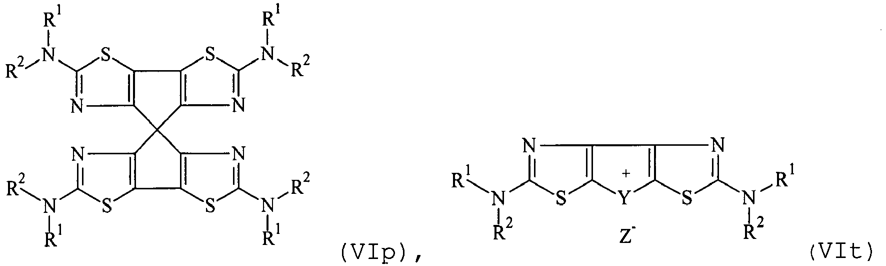 wobei:
- R¹, R², und R³ - unabhängig voneinander - jeweils ein monofunktionelles (Het)arylsystem sind, d.h. ein konjugiertes carbocyclisches oder heterocyclisches Ringsystem, das auch aus linear oder angular anellierten oder verknüpften gleichen oder unterschiedlichen Ringtypen bestehen kann, wobei die peripheren Wasserstoffatome gegebenenfalls durch Alkyl-, Alkoxy-, Dialkylamino- oder Diphenylamiogruppen (Alkyl = C₁ bis C₆) substituiert sein können;
- R⁷ ein Arylensystem ist,
- Z- ein beliebiges Anion ist, insbesondere ein organisches Sulfonat,
- Y gleich -CH oder -N ist;
und
4-substituierte 2-(N,N-Di(het)arylamino)-thiazol-Derivate der folgenden allgemeinen Strukturen VIb, VIf, VIh und VIu: 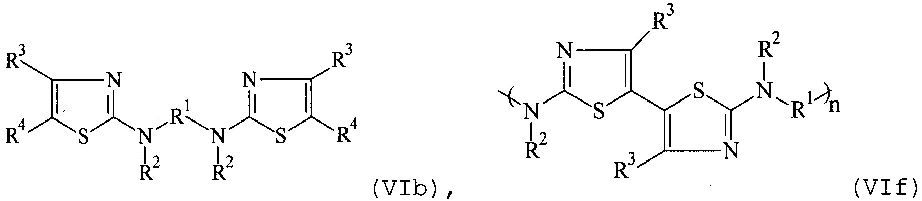 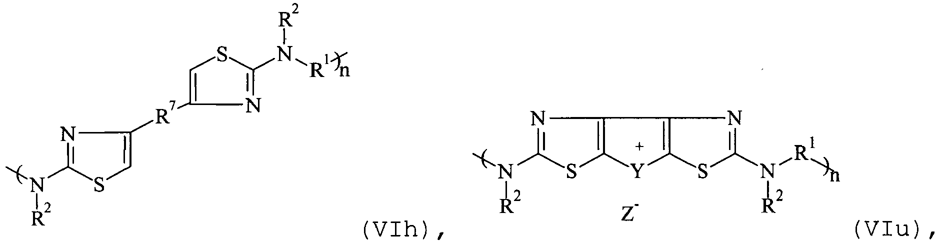 wobei:
- R¹ ein entsprechendes bifunktionelles (Het)arylensystem ist, d.h. ein konjugiertes carbocyclisches oder heterocyclisches Ringsystem, das auch aus linear oder angular anellierten oder verknüpften gleichen oder unterschiedlichen Ringtypen bestehen kann, wobei die peripheren Wasserstoffatome gegebenenfalls durch Alkyl-, Alkoxy-, Dialkylamino- oder Diphenylaminogruppen (Alkyl = C₁ bis C₆) substituiert sein können;
- R² und R³ - unabhängig voneinander - jeweils ein monofunktionelles (Het)arylsystem sind, d.h. ein konjugiertes carbocyclisches oder heterocyclisches Ringsystem, das auch aus linear oder angular anellierten oder verknüpften gleichen oder unterschiedlichen Ringtypen bestehen kann, wobei die peripheren Wasserstoffatome gegebenenfalls durch Alkyl-, Alkoxy-, Dialkylamino- oder Diphenylamiogruppen (Alkyl = C₁ bis C₆) substituiert sein können;
- R⁴ ist 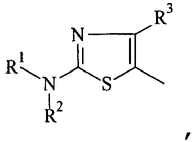 wobei die Reste R¹, R² und R³ innerhalb der Definition des Restes R⁴ den Resten R¹, R² und R³ entsprechen, wie sie für die Strukturen VIe, VIm, VIp und VIt definiert sind;
- R⁷ steht für eine chemische Bindung oder für ein bifunktionelles (Het)arylensystem;
- n eine ganze Zahl von 2 bis 100 bedeutet,
- Z- ein Anion ist, insbesondere ein organisches Sulfonat,
- Y gleich -CH oder -N ist.

Auch offenbart werden 4-substituierte 2-(N,N-Di(het)arylamino)-thiazol-Derivate der allgemeinen Struktur 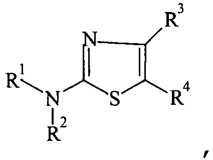 wobei Folgendes gilt:
R¹, R², und R³ sind - unabhängig voneinander - jeweils ein monofunktionelles (Het)arylsystem, d.h. ein konjugiertes carbocyclisches oder heterocyclisches Ringsystem, das auch aus linear oder angular anellierten oder verknüpften gleichen oder unterschiedlichen Ringtypen bestehen kann, wobei die peripheren Wasserstoffatome gegebenenfalls durch Alkyl-, Alkoxy-, Phenoxy-, Dialkylamino- oder Diphenylaminogruppen (Alkyl = C₁ bis C₆) substituiert sein können;
folgende (Het)arylsysteme sind dabei bevorzugt:
   Phenyl-, Biphenyl-, Alkylphenyl-, Alkoxyphenyl-, Phenoxyphenyl-, Naphthyl-, Anthryl-, Phenanthryl-, 4-Tritylphenyl-, Thienyl,- Thiazolyl-, Benzothiazolyl-, Pyridyl-, Chinolyl-, Isochinolyl-, Pyridazinyl-, Pyrimidinyl-, Chinazolyl-, Pyrazinyl-, Chinoxalyl-, Phenazinyl- und Pyrenylsysteme.
R¹ kann in der der allgemeinen Struktur 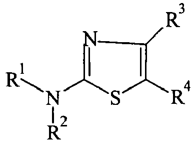 ein entsprechendes bifunktionelles (Het)arylensystem sein, d.h. ein konjugiertes carbocyclisches oder heterocyclisches Ringsystem, das auch aus linear oder angular anellierten oder verknüpften gleichen oder unterschiedlichen Ringtypen bestehen kann, wobei die peripheren Wasserstoffatome gegebenenfalls durch Alkyl-, Alkoxy-, Phenoxy-, Dialkylamino- oder Diphenylaminogruppen (Alkyl = C₁ bis C₆) substituiert sein können;
folgende (Het)arylensysteme sind dabei bevorzugt:
   Phenylen-, Biphenylen-, Alkylphenylen-, Alkoxyphenylen-, Phenoxyphenylen-, Naphthylen-, Anthrylen-, Phenanthrylen-, Thienylen,- Thiazolylen-, Pyridazinylen-, Phthalazinylen- und Pyrazinylensysteme.
R¹ und R² kann in der allgemeinen Struktur 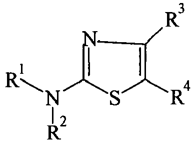 auch gemeinsam eine 10-Phenothiazinyl-, 10-Phenoxazinyl oder 9-Carbazolylgruppierung bilden.
R³ kann in der allgemeinen Struktur 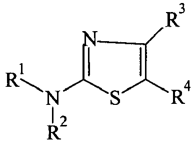 ferner R⁷ bedeuten, wobei R⁷ für eine chemische Bindung oder ein bifunktionelles (Het)arylensystem steht, oder R³ kann in der allgemeinen Struktur 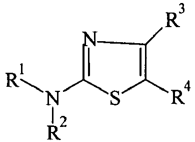 eine der folgenden Gruppierungen sein: 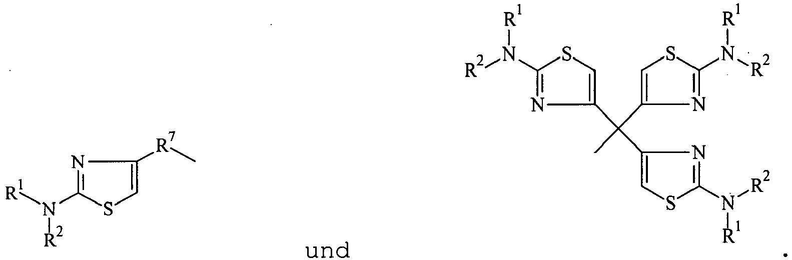
wobei R⁷ für eine chemische Bindung oder ein bifunktionelles (Het)arylensystem steht;
R⁴ kann in der allgemeinen Struktur 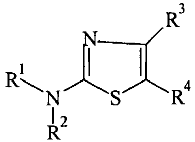 auch H oder 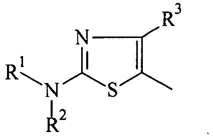 sein;
ferner kann in der allgemeinen Struktur 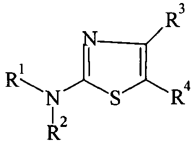 Folgendes der Fall sein: R³ und R⁴ bilden zusammen eine der folgenden Gruppierungen: 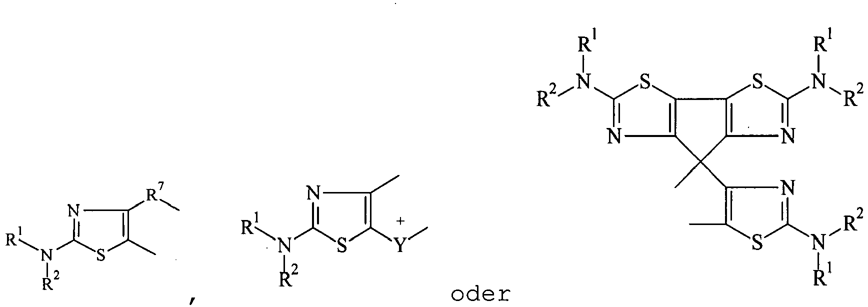
R³ bildet mit R¹ folgendes Polymersegment: 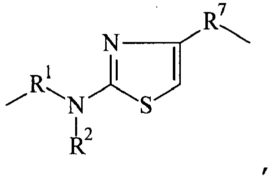
R⁴ bildet mit R¹ folgendes Polymersegment: 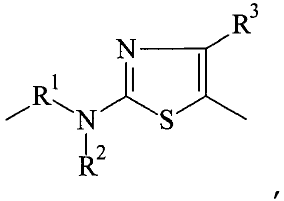
R⁴ und R³ bilden mit R¹ folgende Polymersegmente: 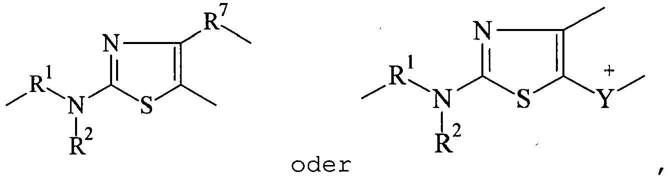
wobei Y jeweils CH oder N bedeutet.

Die Herstellung der Di(het)arylaminothiazol-Derivate erfolgt mittels einer Hetarylringschlussreaktion unter milden Bedingungen und ohne Metallkatalysatoren. Diese Verbindungen können somit in hoher Reinheit hergestellt werden, d.h. die Effizienz dieser Materialien, insbesondere die Elektrolumineszenz, wird nicht durch Verunreinigungen beeinträchtigt, die bei nach bekannten Verfahren hergestellten Verbindungen beispielsweise wegen des eingesetzten Katalysators vorhanden sind. Ein weiterer Vorteil dieser Materialien besteht in verbesserten und für den jeweiligen Zweck anpassbaren Redox-Eigenschaften, bedingt durch die Vielfalt der Strukturmöglichkeiten, die sich aus dem nachfolgend näher geschilderten Aufbauprinzip ergibt. Dieses Aufbauprinzip ermöglicht es ferner, dass die neuen Materialien problemlos auch in Form von Oligomer- und Polymer-Strukturen erhalten werden können, d.h. dass Oligo- und Poly-aminothiazol-Derivate herstellbar sind, wobei der maximale Polymerisationsgrad n = 100 durch die Stufenkondensation nicht überschritten werden kann.

Die Synthese der neuen Verbindungen erfordert zum Teil Vorprodukte, die bislang nicht bekannt sind. Diese Vorprodukte sind aber - aus kommerziell erhältlichen Edukten - in nahezu quantitativer Ausbeute erhältlich.

Darstellung der Thioharnstoffedukte:

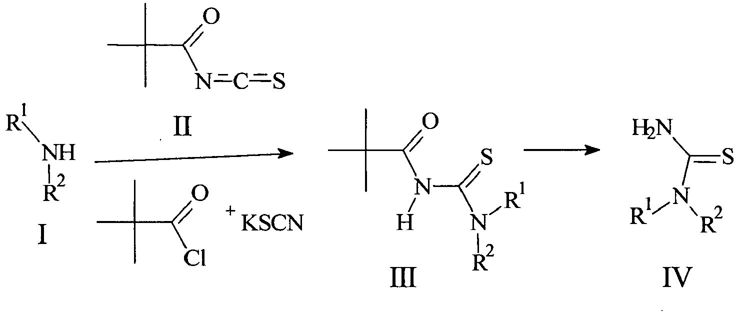

Darstellung der Thiazolderivate:

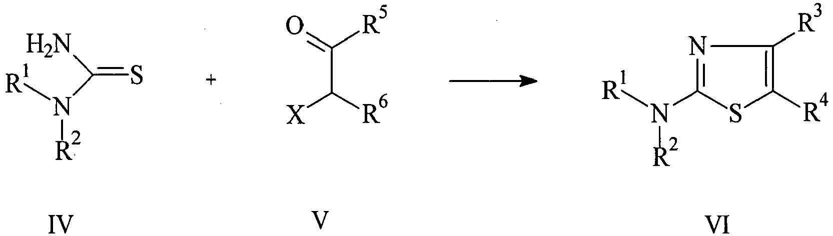

Hierbei gilt Folgendes:
R¹, R², R³ und R⁴ haben die vorstehend angegebene Bedeutung; R⁵ und R⁶ sind - unabhängig voneinander - jeweils ein monofunktionelles (Het)arylsystem, d.h. ein konjugiertes carbocyclisches oder heterocyclisches Ringsystem, das auch aus linear oder angular anellierten oder verknüpften gleichen oder unterschiedlichen Ringtypen bestehen kann, wobei die peripheren Wasserstoffatome gegebenenfalls durch Alkyl-, Alkoxy-, Phenoxy-, Dialkylamino- oder Diphenylaminogruppen (Alkyl = C₁ bis C₆) substituiert sein können;
R⁵ kann ferner die Gruppierung R⁸ sein, wobei die Verbindung V an der freien Bindung eine Gruppe -CO-CH₂X trägt mit X = Halogen, vorzugsweise Cl, Br oder J,
oder R⁵ kann eine Gruppe -C(CO-CH₂X)₃ sein.
R⁶ kann auch Wasserstoff bedeuten.

Die erste Stufe der Synthese der Thiazol-Derivate besteht in der Umsetzung eines sekundären Amins I mit Pivaloylisothiocyanat II zu einem N,N-disubstituierten Pivaloylthioharnstoff III. Die Umsetzung erfolgt in einem geeigneten Lösemittel, vorzugsweise Aceton, bei erhöhter Temperatur, vorzugsweise im Bereich des Siedepunktes des Lösemittels, unter Inertgas. Das Produkt muss in der Regel nicht isoliert werden und wird dann in einer zweiten Stufe in wässriger Salzsäure in den N,N-disubstituierten Thioharnstoff IV überführt.

In einer dritten Stufe wird der N,N-disubstituierte Thioharnstoff IV - je nach Substitutionsmuster (siehe nachfolgend IVa und IVb) - durch Umsetzung mit einer α-Halogen-acylverbindung V in einem geeigneten Lösemittel, vorzugsweise Acetanhydrid, Dimethylformamid oder Ethanol, in ein halbleitendes 4-substituiertes 2-(N,N-Di(het)arylamino)-thiazol-Derivat VI überführt, und zwar je nach Substitutionsmuster der Acyl-verbindung (siehe nachfolgend Vb bis Vd). Dies erfolgt durch eine primäre S-Alkylierung sowie eine nachfolgende Cyclisierung (Ringschlussreaktion) und Aromatisierung entsprechend der "Hantzschen Thiazolsynthese". Die Cyclisierung kann durch Zugabe eines deprotonierend wirkenden Agens, vorzugsweise Triethylamin, beschleunigt werden.

Thiazol-Derivate VI, die in 5-Stellung unsubstituiert sind, d.h. R⁴ = H, können durch oxidative Kupplung in dimere bzw. polymere Derivate VI umgewandelt werden (siehe nachfolgend VIe,f,i,m,n,p,s), die ebenfalls organische Halbleitermaterialien darstellen. Die oxidative Kupplung erfolgt in einem geeigneten Lösemittel, vorzugsweise trockenem Tetrahydrofuran, durch an sich bekannte Oxidationsmittel, vorzugsweise durch Oxidation des jeweiligen - mittels Butyllithium hergestellten - lithiierten Thiazol-Derivates mit Kupfer-II-chlorid oder durch Elektrooxidation auf einem leitfähigen Substrat, beispielsweise auf einer mit ITO beschichteten Glasscheibe (ITO = Indium Tin Oxide).

Mittels halogenierten 1,2-Diketonen (siehe nachfolgend Vd/d) - als α-Halogen-acylverbindung - aus den Thioharnstoffen IV hergestellte Thiazol-Derivate VI, die in 5,5'-Stellung unsubstituiert und in 4,4'-Stellung verbunden sind (siehe nachfolgend VIq und VIr), können mit Orthoameisensäureester oder mit salpetriger Säure, die beispielsweise aus Natriumnitrit oder Isoamylnitrit generiert wird, in entsprechende kationische Lochtransportmaterialien überführt werden (siehe nachfolgend VIt und VIu), die den bekannten Polythiophenen und Polyanilinen in deren Eigenschaft als organische Leiter ähnlich sind.

Nachfolgend sind die Strukturformeln einer größeren Anzahl der neuen Thiazol-Derivate - zusammen mit den unmittelbaren Ausgangsprodukten - in tabellarischer Form wiedergegeben.

| | | | |
|---|---|---|---|
| Organisch halbleitende Produkte aus den Kombinationen von Thioharnstoffen IV und □-Halogen-acylverbindungen V sowie deren Oxidationsprodukte und leitfähige kationische Strukturen | 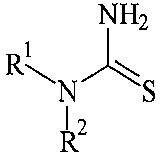 | Oxidation | 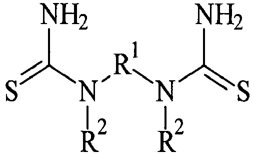 |
| | IVa | | IVb |
| 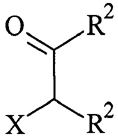 | "nicht relevant" | | 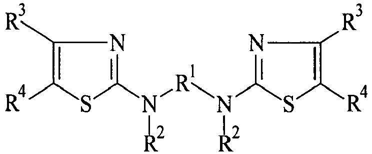 |
| Va | VIa | | VIb |
| | (Vergleichsbeispiel) | | (Ausführungsbeispiel) |
| 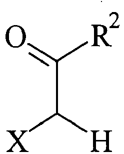 | 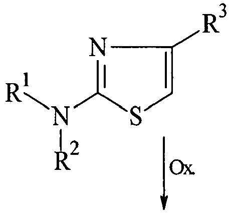 | | 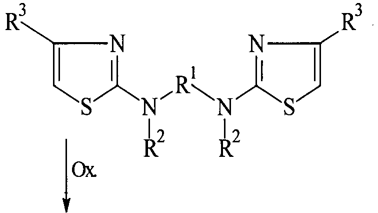 |
| Vb | VIc (Vergleichsbeispiel) | | VId (Vergleichsbeispiel) |
| 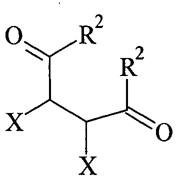 | 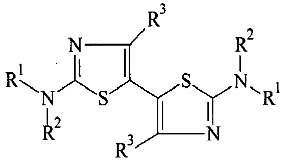 | | 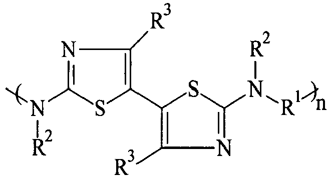 |
| Vc | VIe (Ausführungsbeispiel) | | VIf (Ausführungsbeispiel) |
| 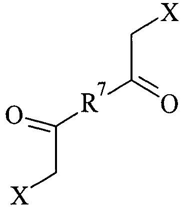 | 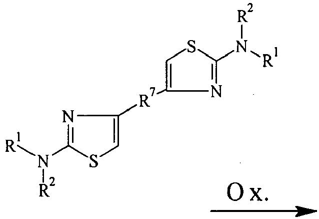 | 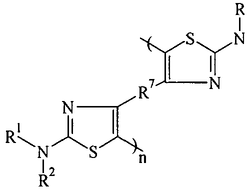 | 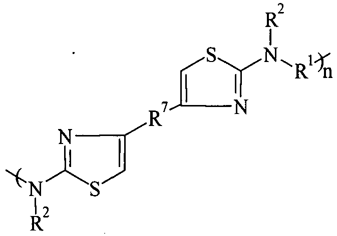 |
| R⁷ = Arylensystem - nicht 1,2-verknüpft Vd/a | VIg (Vergleichsbeispiel) | VIi (Vergleichsbeispiel) | VIh (Ausführungsbeispiel) |
| 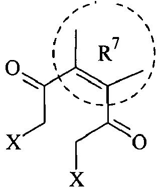 | 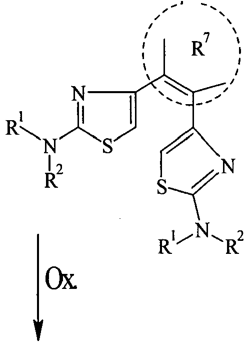 | | 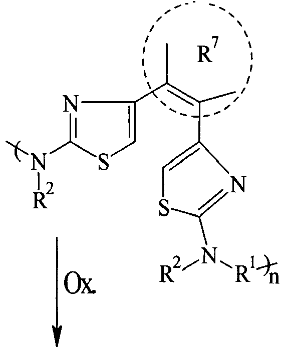 |
| R⁷ = Arylensystem - 1,2-verknüpft Vd/b | VIk (Vergleichsbeispiel) | | VIl (Vergleichsbeispiel) |
| Oxidation | 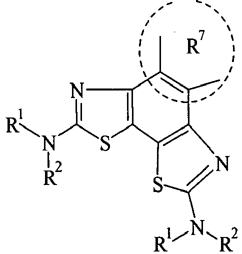 | | 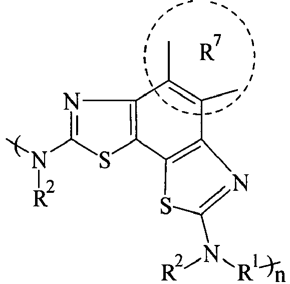 |
| | VIm (Ausführungsbeispiel) | | Vin (Vergleichsbeispiel) |
| 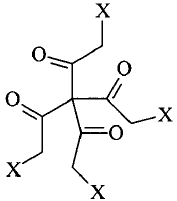 | 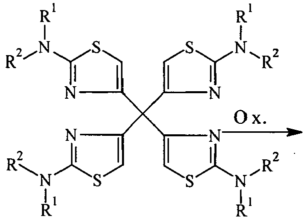 | 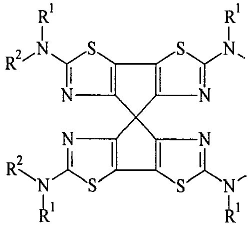 | |
| R⁵ = -C(CO-CH₂X)₃ Vd/c | VIo (Vergleichsbeispiel) | VIp (Ausführungsbeispiel) | |
| 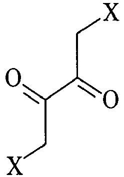 | 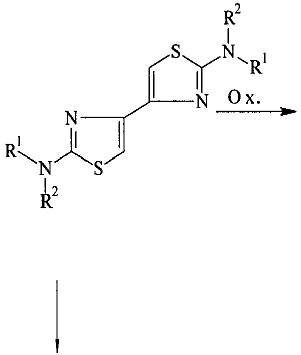 | 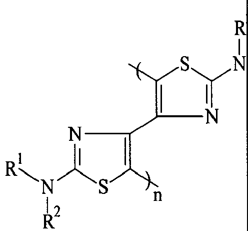 | 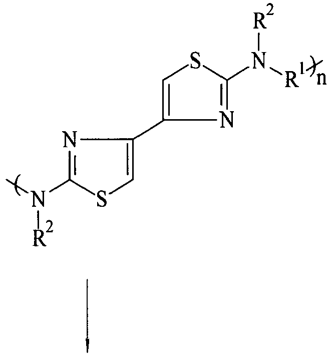 |
| R⁷ = chemische Bindung Vd/d | VIq (Vergleichsbeispiel) | VIs (Vergleichsbeispiel) | VIr (Vergleichsbeispiel) |
| a) CH(OR⁸)₃ | 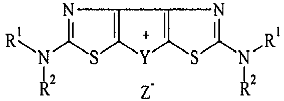 | | 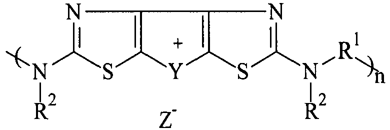 |
| b) HNO₂ | | | |
| | VIt (Ausführungsbeispiel) | | VIu (Ausführungsbeispiel) |

R⁸ ist dabei ein Alkylrest mit 1 bis 5 C-Atomen,
Z- ist ein beliebiges Anion, vorzugsweise ein Polystyrolsulfonat oder ein anderes organisches Sulfonat,
n bedeutet jeweils eine ganze Zahl von 2 bis 100, wobei durchschnittlich ein Polymerisationsgrad n = 20 bis n = 40 erreicht wird.

Die Thiazol-Derivate nach der Erfindung vom Typ VI sind sämtlich geeignete Materialien für den Aufbau von organischen lichtemittierenden Dioden (OLEDs) und organischen photovoltaischen Bauelementen bzw. Zellen. Sie können sowohl in Lochtransportschichten oder Schichtkaskaden als auch in Emitter- und Elektronentransportschichten Verwendung finden. Die jeweilige Schichtposition in OLEDs wird vor allem durch die (Het)aryl- bzw. (Het)arylen-Glieder festgelegt: je mehr dieser Glieder über einen π-Elektronenmangel verfügen, d.h. so genannte π-Unterschussaromaten darstellen, desto besser eignen sich die Thiazol-Derivate als Emitter- und Elektronentransportmaterialien.

Besonders bevorzugt werden folgende Verbindungstypen: - VIb, VId, VIe, VIf, VIg, VIm, VIp, VIt und VIu.

Über den beschriebenen Syntheseweg lassen sich sowohl OLED-Materialien realisieren, die als verdampfbare Verbindungen für so genannte "small molecule devices" geeignet sind, als auch durch Spin-coating verarbeitbare Polymermaterialien für so genannte "polymer devices"; die nicht-polymeren Materialien können dabei ebenfalls durch Spin-coating verarbeitet werden. Wegen der gemeinsamen Grundstruktur sämtlicher Materialien können vorteilhaft auch entsprechende Copolymere mit abgestimmten elektronischen Eigenschaften hergestellt werden. Ferner ist es möglich, die für den jeweiligen Verwendungszweck erforderlichen elektronischen Eigenschaften durch Mischungen entsprechender Materialien zu realisieren, die - aufgrund der strukturellen Ähnlichkeit - sehr gut miteinander verträglich sind. Derart abgestimmte Materialien erlauben daher einen Einschichtaufbau von OLEDs, was sehr vorteilhaft ist. Durch zusätzliche Nutzung der in den Patenten DE 10002423.8 und DE 10002424.6 beschriebenen Materialien für optimale Ladungstransportkaskaden lassen sich OLEDs mit geringerer Betriebsspannung, (onset 2,5 - 3V) realisieren. Bemerkenswert sind auch die im Vergleich zu bekannten carbocyclischen Ladungstransportmaterialien sehr hohen Glasübergangstemperaturen der auf die beschriebene Weise hergestellten Ladungstransport- und Emittermaterialien, die im allgemeinen etwa 50 bis 100°C höher liegen als diejenigen der analogen carbocyclischen Verbindungen, welche bis auf Ausnahmen eine Glasübergangstemperatur zwischen 100°C und 150°C aufweisen. Die Thiazol-Derivate gemäss der Erfindung haben eine Glasübergangstemperatur von mindestens 180°C, bevorzugt von mindestens 200°C, insbesondere im Bereich von 230 bis 250°C.

Anhand von Ausführungsbeispielen soll die Erfindung näher erläutert werden.

### Beispiel 1

### Synthese der Pivaloylthioharnstoffe III

In einem 2 1-Dreihalskolben mit Rückflusskühler, Magnetrührer, Tropftrichter und Inertgasdurchfluss wird 1 mol Kaliumrhodanid in 500ml absolutem Aceton vorgelegt und 1 mol Pivaloylchlorid zugetropft. Danach wird eine Stunde am Rückfluss erhitzt und anschließend die jeweils äquivalente Menge des entsprechenden sekundären Amins I (monofunktionell 1 mol, bifunktionell 0,5 mol), gelöst oder suspendiert in 300ml Aceton, zugetropft. Man erwärmt nun die Reaktionsmischung solange am Rückfluss, bis dünnschicht-chromatografisch kein Amin mehr feststellbar ist. Die Reaktionslösung wird dann abgekühlt und in mindestens die 2fache Menge verdünnte, wässrige Salzsäure eingerührt. Die wässrige Phase wird abgetrennt und verworfen, das Rohprodukt kann sofort weiterverarbeitet werden.

Auf diese Art wird beispielsweise aus N,N'-Diphenyl-1,4-phenylendiamin, Pivaloylchlorid und Kaliumrhodanid N,N'-Diphenyl-N,N'-di(thioform-pivaloylamido)-1,4-phenylendiamin mit einem Schmelzpunkt von 165°C hergestellt.

### Beispiel 2

### Synthese der N,N-disubstituierten Thioharnstoffe IV

In einem Becherglas werden der entsprechende Pivaloylthioharnstoff mit conc.Salzsäure suspendiert. Dabei ist für 1 mol abzuspaltende Pivaloylgruppierung 400 ml conc. Salzsäure zu bemessen. Die Reaktionsmischung wird zum Sieden erhitzt bis die dabei entstehende Schaumbildung wieder beendet ist. Anschließend wird auf Eis gegossen, wobei sich das Produkt abscheidet. Das Produkt wird abgesaugt und aus Ethanol umkristallisiert.

Die Ausbeute beträgt jeweils mindestens 80%.

Auf diese Art wird beispielsweise der Thioharnstoff IVa (R¹ = R² = Phenyl)mit einem Schmelzpunkt von 212°C hergestellt.

### Beispiel 3

### Synthese von Thiazol-Derivaten VI

a) 0,1 mol des jeweiligen N,N-disubstituierten Thioharnstoffs IV. werden in einem Kolben, der mit Rührer und Rückflusskühler versehen ist, zusammen mit der äquivalenten Menge einer α-Halogen-acylverbindung V in 200 ml DMF gelöst, dann wird 1 h auf 100°C erwärmt. Danach werden 0,1 mol Triethylamin zugefügt und es wird weitere 30 min erwärmt. Nach dem Abkühlen wird das gebildete Thiazol-Derivat durch Fällen mit Methanol, in manchen Fällen auch mit Eiswasser, und Absaugen isoliert. Das Rohprodukt wird durch Umkristallisation aus Ethanol gereinigt. Die Ausbeute beträgt jeweils zwischen 60 und 80%.
   Auf diese Art wird beispielsweise das Thiazol-Derivat VIb (R¹ = 1,4-Phenylen und R² = R³ = R⁴ = Phenyl; Vergleichsbeispiel) aus dem bifunktionellen Thioharnsoff IVb (R¹ = 1,4 Phenylen und R² = Phenyl) und Desylchlorid (α-Chlor-α-phenylacetophenon) hergestellt. m.p.= 300°C, MS: MH⁺ = 731, T(g)= 240°C
   ¹H-NMR: (DMF-D₇
   a) 7,65 ppm (s) 4H
   b) 7,35 ppm (d) 4H
   c) 7,62 ppm (d) 4H 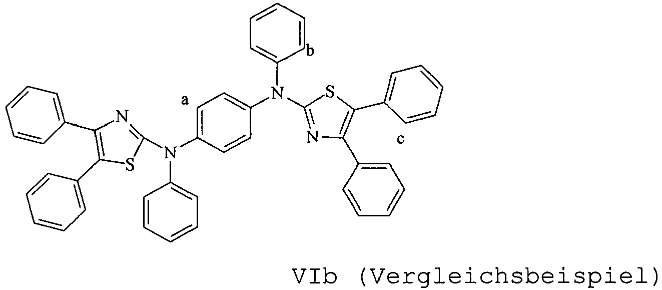
b) Zur Herstellung von polymeren Thiazol-Derivaten VI, beispielsweise VIf (R¹ = 1,4-Phenylen und R² = R³ = Phenyl), wird folgendermaßen vorgegangen:
   0,1 mol bifunktioneller Thioharnstoff IVb (R¹ = 1,4-Phenylen, R² = Phenyl) werden in einem Kolben, der mit Rührer und Rückflusskühler versehen ist, zusammen mit 0,1 mol dimerem Phenacylbromid Vc (R² = Phenyl) in 200 ml DMF gelöst, dann wird 1 h auf 100°C erwärmt. Danach werden - zur Verkappung der Endgruppen - nacheinander ein monofunktioneller Thioharnstoff IVa und ein monofunktionelles Acylhalogenid Va zugegeben, im vorliegenden Fall zunächst 0,01 mol N,N-Diphenylthioharnstoff und nach 60 min 0,01 mol Phenacylbromid. Nach weiteren 60 min werden 0,1 mol Triethylamin zugesetzt und nach weiteren 15 min lässt man abkühlen, wobei das gebildete polymere Thiazol-Derivat ausfällt. Die weitere Aufarbeitung erfolgt in der vorstehend beschriebenen Weise; Ausbeute: ca. 80 %. ¹H-NMR: (DMF-D₇
c) 7,1 ppm (s) 4H
d) 6,9 ppm (t) 2H
c) 7,3 ppm (t) 2H

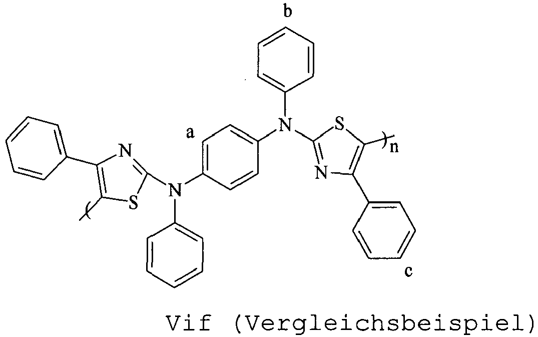

### Beispiel 4

### Synthese von dimeren bzw. polymeren Thiazol-Derivaten VI (durch oxidative Kupplung)

0,01 mol eines in 5-Stellung unsubstituierten Thiazol-Derivates VI werden in einem Kolben, der mit Rückflusskühler, Rührer, Feststoffdosiereinrichtung und Inertgasdurchfluss versehen ist, in 100 ml getrocknetem THF gelöst. Es wird auf -60°C abgekühlt, und dann werden 0,015 mol Butyllithium zugesetzt. Anschließend wird die Kühlung entfernt und man lässt das Reaktionsgemisch bis auf -10°C auftauen. Nachfolgend werden mittels der Feststoffdosiereinrichtung 0,011 mol Kupfer-II-chlorid zugefügt, und dann wird weiter bis auf 40°C erwärmt. Die Reaktion wird nach 30 min abgebrochen, indem das Produkt mit Wasser (bei Polymeren mit Methanol mit einem Zusatz von 10% Wasser) ausgefällt und danach abgesaugt wird. Durch mehrfaches Lösen in THF und Ausfällen mit Methanol wird das Produkt gereinigt. Nicht-polymere Verbindungen können auch durch Sublimation gereinigt werden.

Auf diese Art wird beispielsweise das dimere Thiazol-Derivat **VIe** (R¹ = R² = R³ = Phenyl) aus 2-Diphenylamino-4-phenylthiazol **VIc** hergestellt. M.p.: 255-258°C, MS: MH+=655 sowie das bereits in Beispiel 3b dargestellte Polymer **VIf.**
¹H-NMR: (CDCl₃)
a) 7,21 ppm (d) 8H
b) 7, 68 ppm (d) 4H

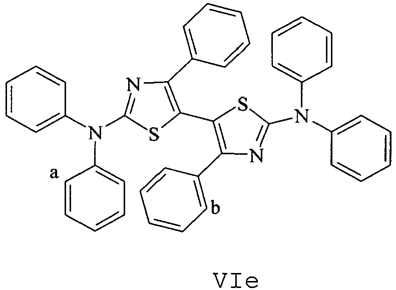

### Beispiel 5

### Synthese von Thiazol-Derivaten VI in Form kationisch leitfähiger Materialien (Y = CH)

0,01 mol eines in 4,4'-Stellung verbundenen und in 5,5'-Stellung unsubstituierten dimeren Thiazol-Derivates VIq bzw. VIr werden in einem Becherglas in 100 ml DMF gelöst und mit 0,015 mol Orthoameisensäuretriethylester versetzt. Nach der Zugabe von 0,01 mol Perchlorsäure und Erwärmen auf ca. 100°C entsteht ein im langwelligen Bereich absorbierendes Farbsalz, welches nach der Zugabe von Ethanol und eventuell etwas Ether ausgefällt und dann abgesaugt wird. Durch Umsetzung des Farbstoff-Perchlorats mit einer Lösung von Natrium-polystyrolsulfonat wird eine wässrige Lösung des jeweiligen kationisch leitfähigen Materials als Polystyrolsulfonat erhalten; diese Lösung kann durch Spin-coating verarbeitet werden.

Auf diese Art wird beispielsweise das Thiazol-Derivat VIt (R¹ = R² = Phenyl und Y = CH) aus dem Thiazol-Derivat VIq (R¹ = R² = Phenyl) und Orthoameisensäure-triethylester in Gegenwart von Perchlorsäure hergestellt.
NMR: (DMF-D₇) :
a) 7, 66 ppm (s) 1H
b) 7,77 ppm (t) 4H

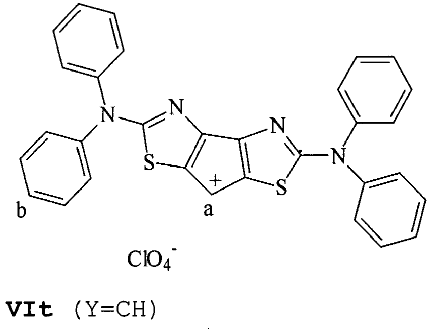

### Beispiel 6

### Synthese von Thiazol-Derivaten VI in Form kationisch leitfähiger Materialien (Y = N)

0,01 mol eines in 4,4'-Stellung verbundenen und in 5,5'-Stellung unsubstituierten dimeren Thiazol-Derivates VIq bzw. VIr werden in einem Becherglas in 100 ml THF gelöst und mit 0,015 mol Isoamylnitrit versetzt. Nach der Zugabe von 0,01 mol Perchlorsäure unter Kühlen und nachfolgendem Erwärmen auf ca. 50°C entsteht ein im langwelligen Bereich absorbierendes Farbsalz, welches nach der Zugabe von Ethanol und eventuell etwas Ether ausgefällt und dann abgesaugt wird. Durch Umsetzung des Farbstoff-Perchlorats mit einer Lösung von Natriumpolystyrolsulfonat wird eine wässrige Lösung des jeweiligen kationisch leitfähigen Materials als Polystyrolsulfonat erhalten; diese Lösung kann durch Spin-coating verarbeitet werden.

Auf diese Art wird beispielsweise das Thiazol-Derivat VIt (R¹ = R² = Phenyl und Y = N) aus dem Thiazol-Derivat VIq (R¹ = R² = Phenyl) und Isoamylnitrit in Gegenwart von Perchlorsäure hergestellt.
NMR: (DMF-D₇) :
a) 7, 66 ppm (s) 1H

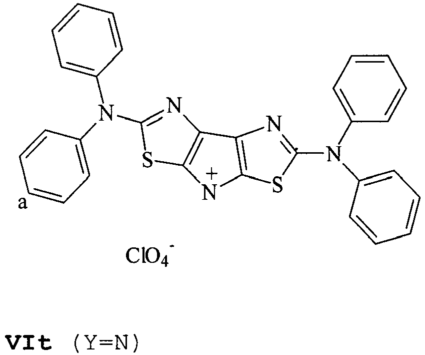

## Patentansprüche

1. 4-substituierte 2-(N,N-Di(het)arylamino)-thiazol-Derivate der folgenden allgemeinen Strukturen VIe, VIm, VIp und VIt: 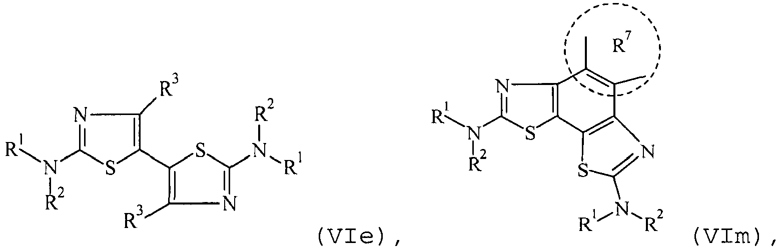 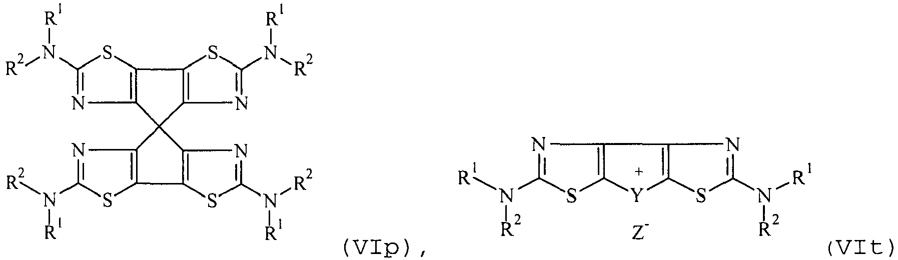 wobei:
- R¹, R², und R³ - unabhängig voneinander - jeweils ein monofunktionelles (Het)arylsystem sind, d.h. ein konjugiertes carbocyclisches oder heterocyclisches Ringsystem, das auch aus linear oder angular anellierten oder verknüpften gleichen oder unterschiedlichen Ringtypen bestehen kann, wobei die peripheren Wasserstoffatome gegebenenfalls durch Alkyl-, Alkoxy-, Dialkylamino- oder Diphenylamiogruppen (Alkyl = C₁ bis C₆) substituiert sein können;
- R⁷ ein Arylensystem ist,
- Z- ein beliebiges Anion ist, insbesondere ein organisches Sulfonat,
- Y gleich -CH oder -N ist;
und
4-substituierte 2-(N,N-Di(het)arylamino)-thiazol-Derivate der folgenden allgemeinen Strukturen VIb, VIf, VIh und VIu: 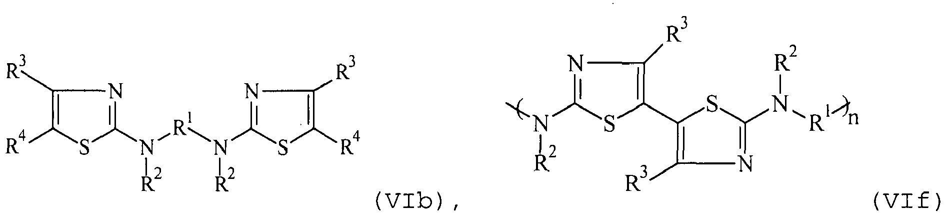 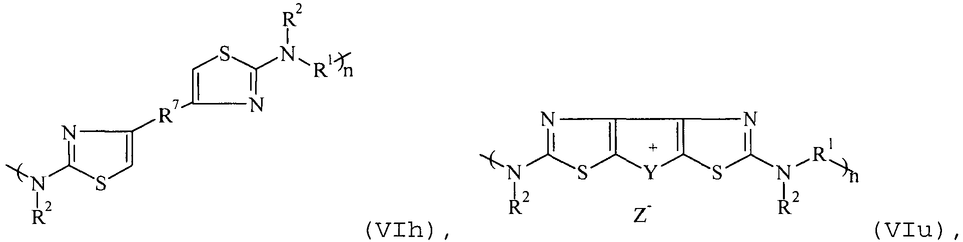 wobei:
- R¹ ein entsprechendes bifunktionelles (Het)arylensystem ist, d.h. ein konjugiertes carbocyclisches oder heterocyclisches Ringsystem, das auch aus linear oder angular anellierten oder verknüpften gleichen oder unterschiedlichen Ringtypen bestehen kann, wobei die peripheren Wasserstoffatome gegebenenfalls durch Alkyl-, Alkoxy-, Dialkylamino- oder Diphenylaminogruppen (Alkyl = C₁ bis C₆) substituiert sein können;
- R² und R³ - unabhängig voneinander - jeweils ein monofunktionelles (Het)arylsystem sind, d.h. ein konjugiertes carbocyclisches oder heterocyclisches Ringsystem, das auch aus linear oder angular anellierten oder verknüpften gleichen oder unterschiedlichen Ringtypen bestehen kann, wobei die peripheren Wasserstoffatome gegebenenfalls durch Alkyl-, Alkoxy-, Dialkylamino- oder Diphenylamiogruppen (Alkyl = C₁ bis C₆) substituiert sein können;
- R⁴ ist 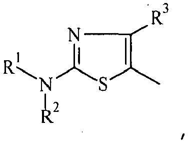 wobei die Reste R¹, R² und R³ innerhalb der Definition des Restes R⁴ den Resten R¹, R² und R³ entsprechen, wie sie für die Strukturen VIe, VIm, VIp und VIt definiert sind;
- R⁷ steht für eine chemische Bindung oder für ein bifunktionelles (Het)arylensystem;
- n eine ganze Zahl von 2 bis 100 bedeutet,
- Z- ein Anion ist, insbesondere ein organisches Sulfonat,
- Y gleich -CH oder -N ist.

2. 4-substituierte 2-(N,N-Di(het)arylamino)-thiazol-Derivate, gemäß Anspruch 1, mit den Strukturen VIe, VIm, VIp und VIt: 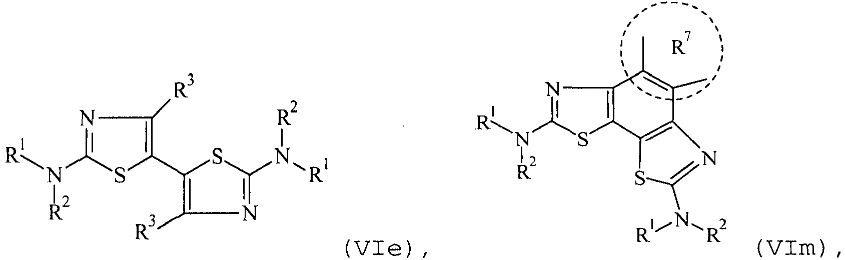 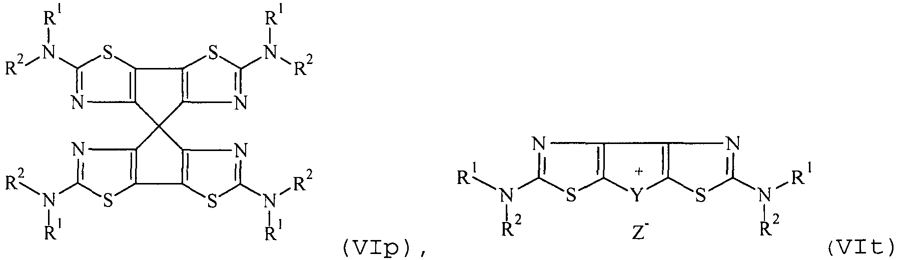 wobei:
- R¹, R² und R³ - unabhängig voneinander - jeweils ein monofunktionelles (Het)arylsystem sind, ausgewählt aus Phenyl-, Biphenyl-, Alkylphenyl-, Alkoxyphenyl-, Naphthyl-, Anthryl-, Phenanthryl-, 4-Tritylphenyl-, Thienyl-, Thiazolyl-, Benzothiazolyl-, Pyridyl-, Chinolyl-, Isochinolyl-, Pyridazinyl-, Pyrimidinyl-, Chinazolyl-, Pyrazinyl-, Chinoxalyl-, Phenazinyl- oder Pyrenylsystemen,
wobei die peripheren Wasserstoffatome gegebenenfalls durch Alkyl-, Alkoxy-, Dialkylamino- oder Diphenylamiogruppen (Alkyl = C₁ bis C₆) substituiert sein können;
- R⁷, Z- und Y definiert sind wie in Anspruch 1.

3. 4-substituierte 2-(N,N-Di(het)arylamino)-thiazol-Derivate, gemäß Anspruch 1, mit den Strukturen VIb, VIf, VIh und VIu: 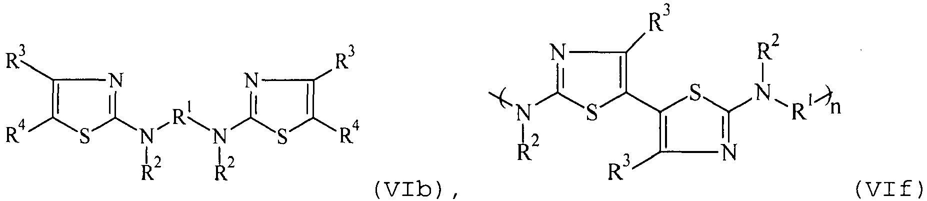 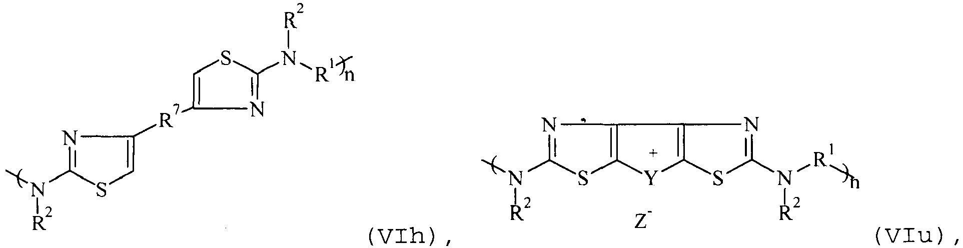 wobei:
- R¹ ein entsprechendes bifunktionelles (Het)arylensystem ist, ausgewählt aus Phenylen-, Biphenylen-, Alkylphenylen-, Alkoxyphenylen-, Naphthylen-, Anthrylen-, Phenanthrylen-, Thienylen-, Thiazolylen-, Pyridazinylen-, Phthalazinylen-, und Pyrazinylensystemen, wobei die peripheren Wasserstoff atome gegebenenfalls durch Alkyl-, Alkoxy-, Dialkylamino- oder Diphenylaminogruppen (Alkyl = C₁ bis C₆) substituiert sein können;
- R² und R³ - unabhängig voneinander - jeweils ein monofunktionelles (Het)arylsystem sind, ausgewählt aus Phenyl-, Biphenyl-, Alkylphenyl-, Alkoxyphenyl-, Naphthyl-, Anthryl-, Phenanthryl-, 4-Tritylphenyl-, Thienyl-, Thiazolyl-, Benzothiazolyl-, Pyridyl-, Chinolyl-, Isochinolyl-, Pyridazinyl-, Pyrimidinyl-, Chinazolyl-, Pyrazinyl-, Chinoxalyl-, Phenazinyl- oder Pyrenylsystemen, wobei die peripheren Wasserstoffatome gegebenenfalls durch Alkyl-, Alkoxy-, Dialkylamino- oder Diphenylamiogruppen (Alkyl = C₁ bis C₆) substituiert sein können;
- R⁴, R⁷, n, Z- und Y definiert sind wie in Anspruch 1.

4. Verfahren zur Herstellung von Thiazol-Derivaten nach Anspruch 1, **gekennzeichnet durch** folgende Schritte:
(a) Umsetzung eines sekundären Amins der Formel R¹-NH-R² mit einem Pivaloylisothiocyanat zu einem Pivaloylthioharnstoff;
(b) Überführung des Pivaloylthioharnstoffs in einen Thioharnstoff der Formel H₂N-C(=S)-NR¹R²;
(c) Umsetzung des Thioharnstoffs mit einer (het)aryl-substituierten α-Halogen-acyl-Verbindung der Formel O=C(R⁵)-C(R⁶)-X zu einem 2-(N,N-Di(het)arylamino)-4-(het)aryl-thiazol-Derivat oder einem 2-(N,N-Di(het)arylamino)-4,5-di(het)aryl-thiazol-Derivat,
wobei die Reste R¹ bis R² definiert sind wie in Anspruch 1 und wobei gilt:
R⁵ und R⁶ sind - unabhängig voneinander - jeweils ein monofunktionelles (Het)arylsystem,
R⁵ kann ferner aus R⁸, wobei R⁸ ein Alkylrest mit 1 bis 5 C-Atomen umfasst, und der Gruppe -C(CO-CH₂X)₃ ausgewählt sein,
X ist ein Halogen
R⁶ umfasst auch Wasserstoff.

5. Verfahren nach Anspruch 4, **dadurch gekennzeichnet, dass** die 2-(N,N-Di(het)arylamino)-4-(het)aryl-thiazol-Derivate oxidativ in ein Dimer oder Polymer überführt werden.

6. Verfahren nach Anspruch 4, **dadurch gekennzeichnet, dass** die 4,4'-Bisthiazolyl-Derivate 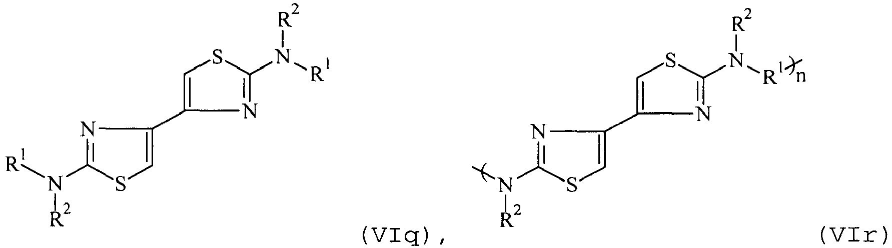 mittels Orthoameisensäureester oder salpetriger Säure in die heterocyclischen, kationischen Polymethine oder Polyazamethine 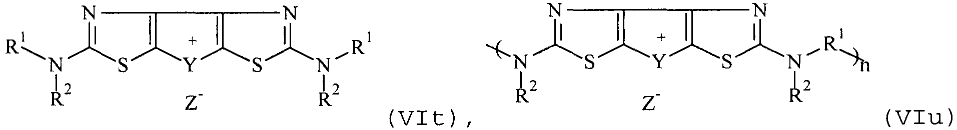 überführt werden, wobei Z und n wie in Anspruch 1 definiert sind.

7. Verwendung der Thiazol-Derivate nach den Ansprüchen 1 bis 3 in organischen lichtemittierenden Dioden.

8. Verwendung der Thiazol-Derivate nach den Ansprüchen 1 bis 3 in organischen photovoltaischen Bauelementen.

9. Organische lichtemittierende Diode, die zumindest ein 4-substituiertes 2-(N,N-Di(het)arylamino)-thiazol-Derivat der allgemeinen Strukturen VIb, VIe, VIf, VIh, VIm, VIp, VIt und VIu nach Anspruch 1 als Ladungstransportschicht und/oder Emitterschicht umfasst, wobei die elektronischen Eigenschaften der (Het)arylsubstituenten bestimmen, ob das Thiazol-Derivat elektronentransportierend oder löchertransportierend wirkt.

10. Thiazol-Derivat nach Anspruch 1 mit einer Glasübergangstemperatur (Tg) von mindestens 180°C.

## Claims

1. 4-Substituted 2-(N,N-di(het)arylamino)thiazole derivatives of the following general structures VIe, VIm, VIp and VIt: 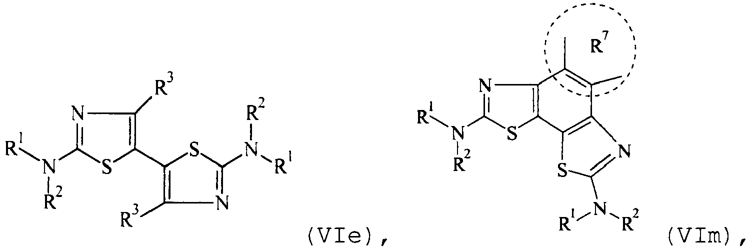 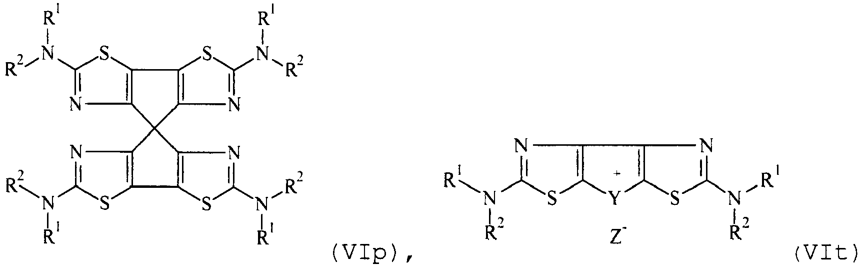 wherein:
- R¹, R² and R³ are, mutually independently, in each case a monofunctional (het)aryl system, i.e. a conjugated carbocyclic or heterocyclic ring system which may also consist of linearly or angularly anellated or linked identical or different ring types, wherein the peripheral hydrogen atoms may optionally be substituted by alkyl, alkoxy, dialkylamino or diphenylamio groups (alkyl = C₁ to C₆) ;
- R⁷ is an arylene system,
- Z- is any desired anion, in particular an organic sulfonate,
- Y is -CH or -N;
and
4-substituted 2-(N,N-di(het)arylamino)thiazole derivatives of the following general structures VIb, VIf, VIh and VIu: 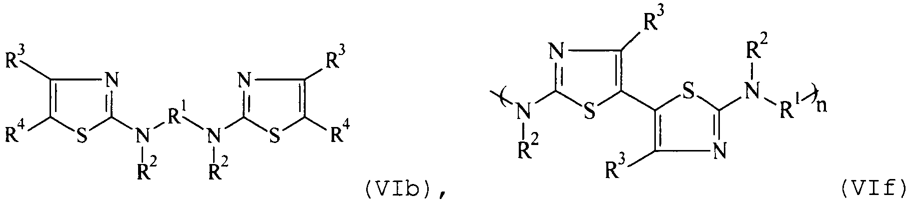 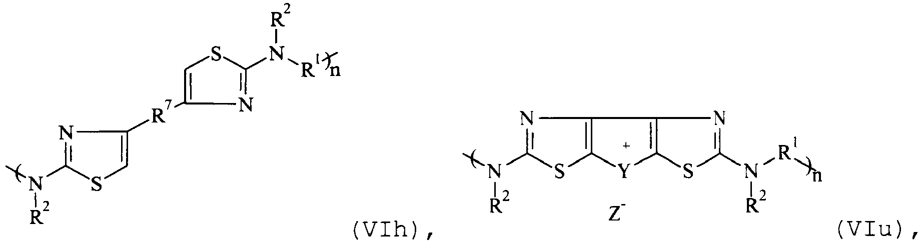 wherein:
- R¹ is a corresponding difunctional (het)arylene system, i.e. a conjugated carbocyclic or heterocyclic ring system which may also consist of linearly or angularly anellated or linked identical or different ring types, wherein the peripheral hydrogen atoms may optionally be substituted by alkyl, alkoxy, dialkylamino or diphenylamino groups (alkyl = C₁ to C₆) ;
- R² and R³ are, mutually independently, in each case a monofunctional (het)aryl system, i.e. a conjugated carbocyclic or heterocyclic ring system which may also consist of linearly or angularly anellated or linked identical or different ring types, wherein the peripheral hydrogen atoms may optionally be substituted by alkyl, alkoxy, dialkylamino or diphenylamio groups (alkyl = C₁ to C₆);
- R⁴ is 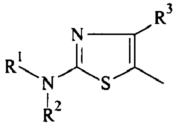 wherein, within the definition of residue R⁴, the residues R¹ R² and R³ correspond to residues R¹, R² and R³ as defined for the structures VIe, VIm, VIp and VIt;
- R⁷ denotes a chemical bond or a difunctional (het)arylene system;
- n means an integer from 2 to 100,
- Z- is an anion, in particular an organic sulfonate,
- Y is -CH or -N.

2. 4-Substituted 2-(N,N-di(het)arylamino)thiazole derivatives according to claim 1 having the structures VIe, VIm, VIp and VIt: 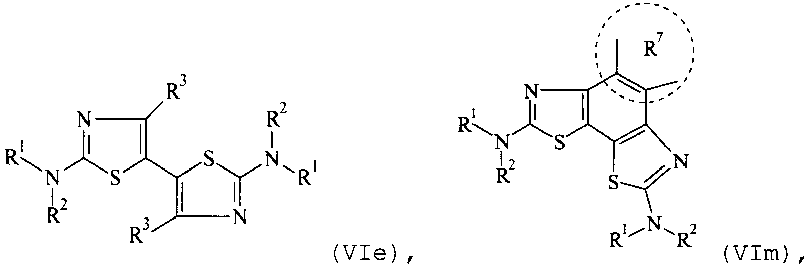 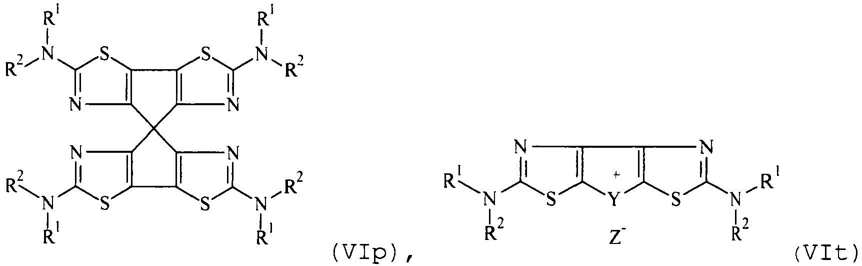 wherein:
- R¹, R² and R³ are, mutually independently, in each case a monofunctional (het)aryl system selected from phenyl, biphenyl, alkylphenyl, alkoxyphenyl, naphthyl, anthryl, phenanthryl, 4-tritylphenyl, thienyl, thiazolyl, benzothiazolyl, pyridyl, quinolyl, isoquinolyl, pyridazinyl, pyrimidinyl, quinazolyl, pyrazinyl, quinoxalyl, phenazinyl or pyrenyl systems,
wherein the peripheral hydrogen atoms may optionally be substituted by alkyl, alkoxy, dialkylamino or diphenylamio groups (alkyl = C₁ to C₆) ;
- R⁷, Z- and Y are defined as in claim 1.

3. 4-Substituted 2-(N,N-di(het)arylamino)thiazole derivatives according to claim 1 having the structures VIb, VIf, VIh and VIu: 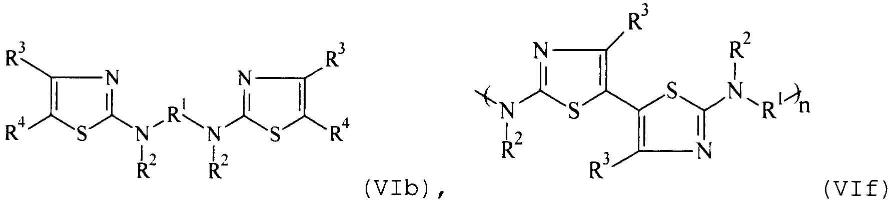 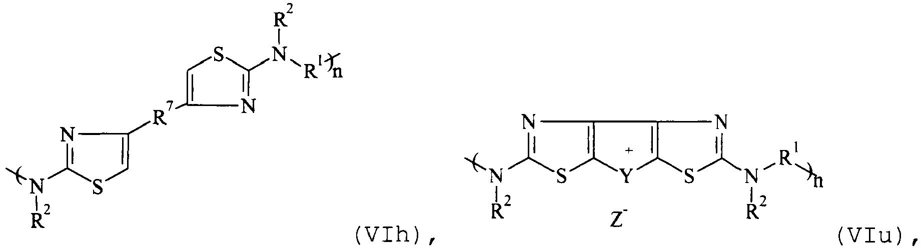 wherein:
- R¹ is a corresponding difunctional (het)arylene system selected from phenylene, biphenylene, alkylphenylene, alkoxyphenylene, naphthylene, anthrylene, phenanthrylene, thienylene, thiazolylene, pyridazinylene, phthalazinylene and pyrazinylene systems, wherein the peripheral hydrogen atoms may optionally be substituted by alkyl, alkoxy, dialkylamino or diphenylamino groups (alkyl = C₁ to C₆);
- R² and R³ are, mutually independently, in each case a monofunctional (het)aryl system, selected from phenyl, biphenyl, alkylphenyl, alkoxyphenyl, naphthyl, anthryl, phenanthryl, 4-tritylphenyl, thienyl, thiazolyl, benzothiazolyl, pyridyl, quinolyl, isoquinolyl, pyridazinyl, pyrimidinyl, quinazolyl, pyrazinyl, quinoxalyl, phenazinyl or pyrenyl systems, wherein the peripheral hydrogen atoms may optionally be substituted by alkyl, alkoxy, dialkylamino or diphenylamio groups (alkyl = C₁ to C₆);
- R⁴, R⁷, n, Z- and Y are defined as in claim 1.

4. Method for producing thiazole derivatives according to claim 1, **characterized by** the following steps:
(a) reacting a secondary amine of formula R¹-NH-R² with a pivaloyl isothiocyanate to yield a pivaloyl thiourea;
(b) converting the pivaloyl thiourea into a thiourea of formula H₂N-C(=S)-NR¹R²;
(c) reacting the thiourea with a (het)aryl-substituted α-haloacyl compound of formula O=C(R⁵)-C(R⁶)-X to yield a 2-(N,N-di(het)arylamino)-4-(het)arylthiazole derivative or a 2-(N,N-di(het)arylamino)-4,5-di(het)arylthiazole derivative,
wherein residues R¹ to R² are defined as in claim 1 and wherein:
R⁵ and R⁶ are, mutually independently, in each case a monofunctional (het)aryl system,
R⁵ may furthermore be selected from R⁸, wherein R⁸ comprises an alkyl residue with 1 to 5 C atoms, and the group -C(CO-CH₂X)₃,
X is a halogen,
R⁶ also comprises hydrogen.

5. Method according to claim 4, **characterized in that** the 2-(N,N-di(het)arylamino)-4-(het)arylthiazole derivatives are oxidatively converted into a dimer or polymer.

6. Method according to claim 4, **characterized in that** the 4,4'-bisthiazolyl derivatives 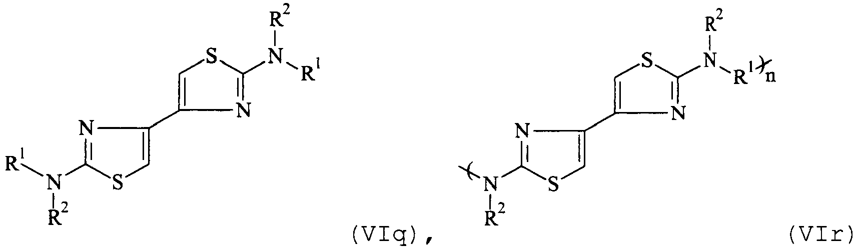 are converted by means of orthoformic acid esters or nitrous acid into the heterocyclic, cationic polymethines or polyazamethines 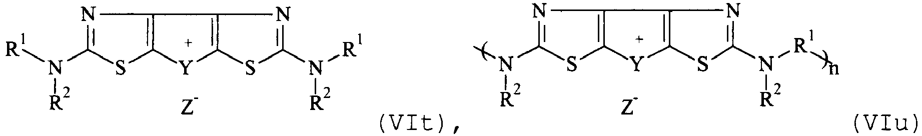 wherein Z and n are defined as in claim 1.

7. Use of the thiazole derivatives according to claims 1 to 3 in organic light-emitting diodes.

8. Use of the thiazole derivatives according to claims 1 to 3 in organic photovoltaic devices.

9. Organic light-emitting diode which comprises at least one in 4-substituted 2-(N,N-di(het)arylamino)thiazole derivative of the general structures VIb, VIe, VIf, VIh, VIm, VIp, VIt and VIu according to claim 1 as a charge transport layer and/or emitter layer, wherein the electronic properties of the (het)aryl substituents determine whether the thiazole derivative acts as an electron transporter or a hole transporter.

10. Thiazole derivative according to claim 1 with a glass transition temperature (Tg) of at least 180°C.

## Revendications

1. Dérivés substitués en 4ème position de 2-(N,N-di(hét)arylamino)-thiazole présentant les structures générales suivantes VIe, VIm, VIp et VIt : 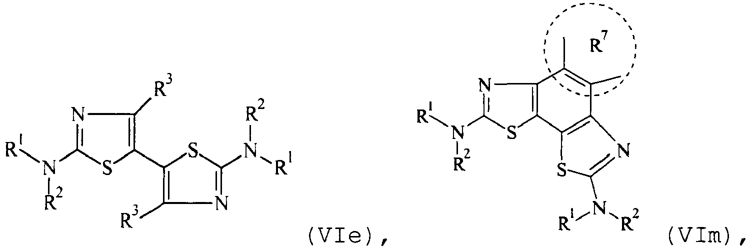 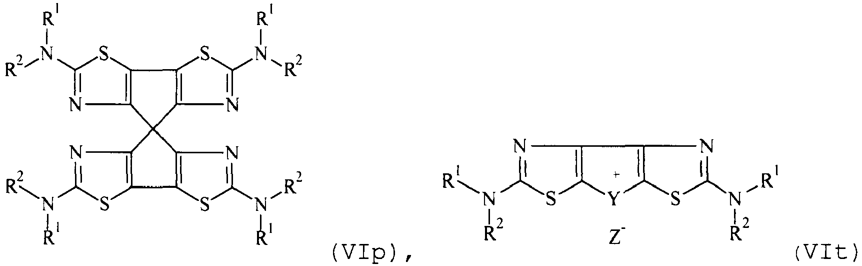 dans lesquelles :
- R¹, R² et R³ - indépendamment les uns des autres - représentent à chaque fois un système (hét)aryle monofonctionnel, c'est-à-dire un système cyclique carboxylique ou hétérocyclique conjugué, qui peut également être constitué par des types de cycles identiques ou différents, annelés ou liés de manière linéaire ou angulaire, les atomes d'hydrogène périphériques pouvant le cas échéant être substitués par des groupes alkyle, alcoxy, dialkylamino ou diphénylamio (alkyle = C₁ à C₆) ;
- R⁷ représente un système arylène,
- Z- représente un anion quelconque, en particulier un sulfonate organique
- Y représente -CH ou -N ;
et
dérivés substitués en 4ème position de 2-(N,N-di(hét)arylamino)-thiazole présentant les structures générales suivantes VIb, VIf, VIh et VIu : 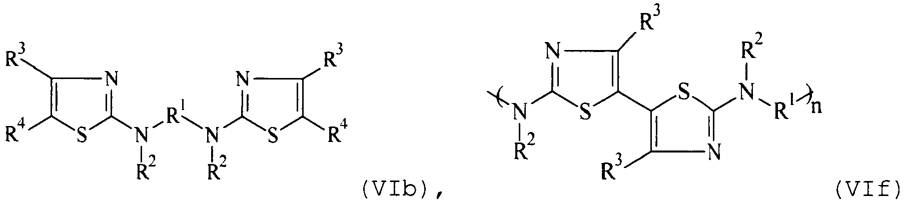 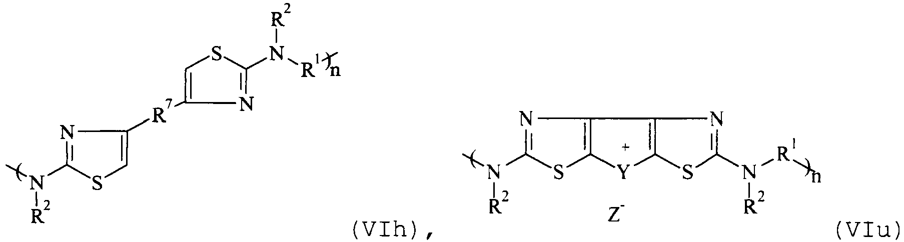 dans lesquelles :
- R¹ représente un système (hét)arylène bifonctionnel correspondant, c'est-à-dire un système cyclique carboxylique ou hétérocyclique conjugué, qui peut également être constitué par des types de cycles identiques ou différents, annelés ou liés de manière linéaire ou angulaire, les atomes d'hydrogène périphériques pouvant le cas échéant être substitués par des groupes alkyle, alcoxy, dialkylamino ou diphénylamino (alkyle = C₁ à C₆) ;
- R² et R³ - indépendamment l'un de l'autre - représentent à chaque fois un système (hét)aryle monofonctionnel, c'est-à-dire un système cyclique carboxylique ou hétérocyclique conjugué, qui peut également être constitué par des types de cycles identiques ou différents, annelés ou liés de manière linéaire ou angulaire, les atomes d'hydrogène périphériques pouvant le cas échéant être substitués par des groupes alkyle, alcoxy, dialkylamino ou diphénylamio (alkyle = C₁ à C₆) ;
- R⁴ représente 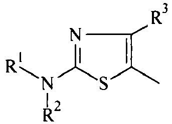 les radicaux R¹ R² et R³ correspondant, dans la définition du radical R⁴, aux radicaux R¹, R² et R³ tels qu'ils sont définis pour les structures VIe, VIm, VIp et VIt ;
- R⁷ représente une liaison chimique ou un système (hét)arylène bifonctionnel ;
- n signifie un nombre entier de 2 à 100,
- Z- représente un anion, en particulier un sulfonate organique,
- Y représente -CH ou -N.

2. Dérivés substitués en 4ème position de 2-(N,N-di(hét)arylamino)-thiazole selon la revendication 1, présentant les structures VIe, VIm, VIp et VIt : 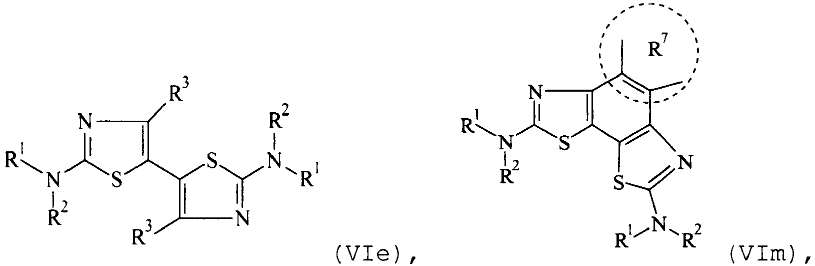 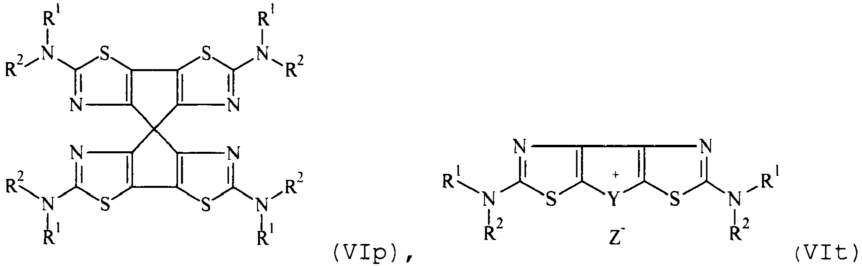 dans lesquelles :
- R¹, R² et R³ - indépendamment les uns des autres - représentent à chaque fois un système (hét)aryle monofonctionnel, choisi parmi les systèmes phényle, biphényle, alkylphényle, alcoxyphényle, naphtyle, anthryle, phénanthryle, 4-tritylphényle, thiényle, thiazolyle, benzothiazolyle, pyridyle, quinoléyle, isoquinoléyle, pyridazinyle, pyrimidinyle, quinazolyle, pyrazinyle, quinoxalyle, phénazinyle ou pyrényle, les atomes d'hydrogène périphériques pouvant le cas échéant être substitués par des groupes alkyle, alcoxy, dialkylamino ou diphénylamio (alkyle = C₁ à C₆) ;
- R⁷, Z- et Y sont définis comme dans la revendication 1.

3. Dérivés substitués en 4ème position de 2-(N,N-di(hét)arylamino)-thiazole selon la revendication 1, présentant les structures VIb, VIf, VIh et VIu : 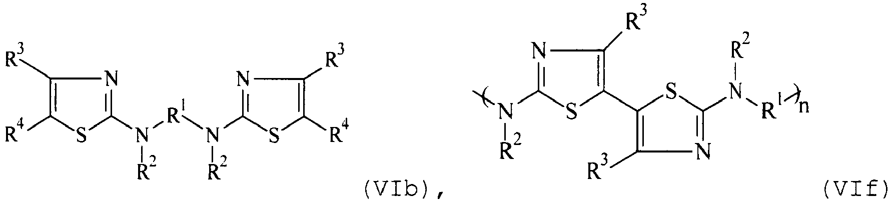 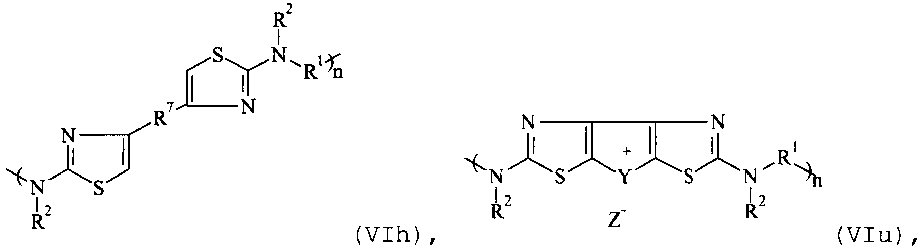 dans lesquelles :
- R¹ représente un système (hét)arylène bifonctionnel correspondant, choisi parmi les systèmes phénylène, biphénylène, alkylphénylène, alcoxyphénylène, naphtylène, anthrylène, phénanthrylène, thiénylène, thiazolylène, pyridazinylène, phtalazinylène et pyrazinylène, les atomes d'hydrogène périphériques pouvant le cas échéant être substitués par des groupes alkyle, alcoxy, dialkylamino ou diphénylamino (alkyle = C₁ à C₆) ;
- R² et R³ - indépendamment l'un de l'autre - représentent à chaque fois un système (hét)aryle monofonctionnel, choisi parmi les systèmes phényle, biphényle, alkylphényle, alcoxyphényle, naphtyle, anthryle, phénanthryle, 4-tritylphényle, thiényle, thiazolyle, benzothiazolyle, pyridyle, quinoléyle, isoquinoléyle, pyridazinyle, pyrimidinyle, quinazolyle, pyrazinyle, quinoxalyle, phénazinyle ou pyrényle,
les atomes d'hydrogène périphériques pouvant le cas échéant être substitués par des groupes alkyle, alcoxy, dialkylamino ou diphénylamio (alkyle = C₁ à C₆) ;
- R⁴, R⁷, n, Z- et Y sont définis comme dans la revendication 1.

4. Procédé pour la préparation de dérivés de thiazole selon la revendication 1, **caractérisé par** les étapes suivantes :
(a) transformation d'une amine secondaire de formule R¹-NH-R² avec un isothiocyanate de pivaloyle en une pivaloylthio-urée ;
(b) transformation de la pivaloylthio-urée en une thio-urée de formule H₂N-C(=S)-NR¹R² ;
(c) transformation de la thio-urée avec un composé α-halogéno-acyle substitué par (hét)aryle de formule O=C(R⁵)-C(R⁶)-X en un dérivé de 2-(N,N-di(hét)arylamino)-4-(hét)arylthiazole ou en un dérivé de 2-(N,N-di(hét)arylamino)-4,5-di(hét)arylthiazole,
les radicaux R¹ et R² étant définis comme dans la revendication 1 et où :
R⁵ et R⁶ représentent - indépendamment l'un de l'autre - à chaque fois un système (hét)aryle monofonctionnel,
R⁵ peut en outre être choisi parmi R⁸, R⁸ comprenant un radical alkyle présentant 1 à 5 atomes de carbone, et le groupe -C(CO-CH₂X)₃,
X représente un halogène
R⁶ comprend également l'hydrogène.

5. Procédé selon la revendication 4, **caractérisé en ce que** les dérivés de 2-(N,N-di(hét)arylamino)-4-(hét)arylthiazole sont transformés par oxydation en un dimère ou un polymère.

6. Procédé selon la revendication 4, **caractérisé en ce que** les dérivés 4,4'-bisthiazolyle 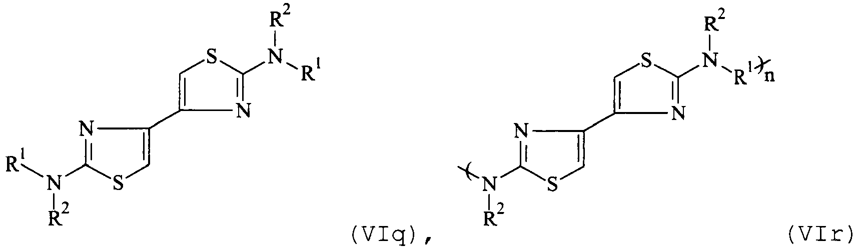 sont transformés au moyen d'acide orthoformique ou d'acide nitreux en polyméthines ou poly-azaméthines hétérocycliques, cationiques 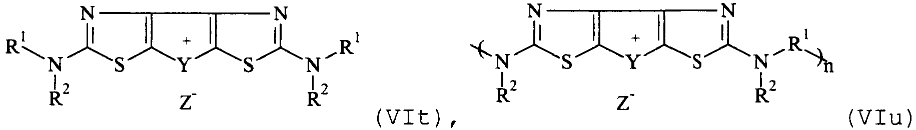 Z et n étant définis comme dans la revendication 1.

7. Utilisation des dérivés de thiazole selon les revendications 1 à 3 dans des diodes électroluminescentes organiques.

8. Utilisation des dérivés de thiazole selon les revendications 1 à 3 dans des éléments photovoltaïques organiques.

9. Diode électroluminescente organique, qui comprend au moins un dérivé substitué en 4ème position de 2-(N,N-di(hét)arylamino)-thiazole des structures générales VIb, VIe, VIf, VIh, VIm, VIp, VIt et VIu selon la revendication 1 comme couche de transport de charges et/ou couche émettrice, les propriétés électroniques du substituant (hét)aryle déterminant le fait que le dérivé de thiazole agit par transport d'électrons ou par transport de trous.

10. Dérivé de thiazole selon la revendication 1 présentant une température de transition vitreuse (Tg) d'au moins 180°C.
